(19) Europäisches Patentamt
European Patent Office
Office européen des brevets

(11) Numéro de publication : **0 463 946 A1**

(12) **DEMANDE DE BREVET EUROPEEN**

(21) Numéro de dépôt : **91401683.7**

(22) Date de dépôt : **21.06.91**

(51) Int. Cl.[5] : **C07C 47/575,** C07C 45/64, C07C 41/16

(30) Priorité : **27.06.90 FR 9008071**
**30.11.90 FR 9015000**

(43) Date de publication de la demande :
**02.01.92 Bulletin 92/01**

(84) Etats contractants désignés :
**DE FR GB IT NL**

(71) Demandeur : **RHONE-POULENC CHIMIE**
**25, quai Paul Doumer**
**F-92408 Courbevoie Cédex (FR)**

(72) Inventeur : **Huet, Michel**
**Flat 14, Mulberry Close, Off Coslany Street**
**Norwich NR3 3PJ (GB)**
Inventeur : **Nobel, Dominique**
**132, allée du Chateau**
**F-69270 Fontaines St Martin (FR)**

(74) Mandataire : **Dutruc-Rosset, Marie-Claude et al**
**RHONE-POULENC CHIMIE Service Brevets Chimie 25, Quai Paul Doumer**
**F-92408 Courbevoie Cédex (FR)**

(54) **Procédé de préparation de composés aromatiques polyalkoxylés.**

(57) La présente invention a pour objet un procédé de préparation de composés aromatiques polyalkoxylés, à partir de composés aromatiques porteurs d'au moins un atome d'halogène et d'au moins un groupe hydroxyle.

Plus précisément, l'invention est un procédé de préparation de composés aromatiques polyalkoxylés caractérisé par le fait que l'on effectue les étapes suivantes :

1 - On fait réagir un composé aromatique porteur d'au moins un atome d'halogène et d'au moins un groupe hydroxyle avec un alcoolate de métal alcalin ou alcalino-terreux, en présence d'un catalyseur qui est le cuivre et/ou un composé du cuivre et d'un co-catalyseur choisi parmi les carbonates organiques, les carbonates mixtes organo-métalliques, le dioxyde de carbone ou les composés susceptibles de former du dioxyde de carbone.

2 - On réalise la O-alkylation du ou des groupes hydroxyle du produit obtenu précédemment après alkoxylation, par addition directement dans le milieu réactionnel organique d'un agent d'alkylation choisi parmi les halogénures d'alkyle inférieurs, les dialkylsulfates, les dialkylcarbonates.

L'invention permet ainsi de réaliser à la suite, la réaction d'alkoxylation et la réaction de O-alkylation.

EP 0 463 946 A1

La présente invention a pour objet un procédé de préparation de composés aromatiques polyalkoxylés, à partir de composés aromatiques porteurs d'au moins un atome d'halogène et d'au moins un groupe hydroxyle et, plus particulièrement, de dialkoxy-3,4 benzaldéhyde et de trialkoxy-3,4,5 benzaldéhydes.

L'invention vise plus particulièrement la préparation de triméthoxy-3,4,5 benzaldéhyde à partir de bromo-5 hydroxy-4 méthoxy-3 benzaldéhyde dénommé couramment "bromo-5 vanilline" et du diméthoxy-3,4 benzaldéhyde appelé "vératraldéhyde".

Une voie classique de préparation de triméthoxy-3,4,5 benzaldéhyde à partir de bromo-5 vanilline consiste à hydrolyser l'atome de brome à l'aide d'une solution aqueuse de soude et en présence de cuivre.

Après isolement du dihydroxy-4,5 méthoxy-3 benzaldéhyde (ou hydroxy-5 vanilline), celui-ci conduit au triméthoxy-3,4,5 benzaldéhyde après méthylation à l'aide de sulfate de diméthyle ou chlorure de méthyle.

Ainsi le brevet FR-A 2 177 693 décrit la méthylation de l'hydroxy-5 vanilline par du sulfate de diméthyle, en présence d'un carbonate de métal alcalin.

L'hydroxy-5 vanilline est elle-même obtenue par hydrolyse de la bromo-5 vanilline puis est extraite, notamment, par le toluène pendant 47 heures, recristallisée, lavée et séchée.

La phase d'isolement de l'hydroxy-5 vanilline est donc longue et coûteuse. En outre, le catalyseur ne peut pas être récupéré et recyclé.

Avant d'éviter la séparation intermédiaire de l'hydroxy-5 vanilline, on a proposé selon le brevet FR-A 2 609 984, un procédé de préparation de dialkoxy-4,5 méthoxy-3 benzaldéhyde à partir de bromo-5 vanilline qui consiste à effectuer successivement, l'hydrolyse de la bromo-5 vanilline en hydroxy-5 vanilline, par un hydroxyde de métal alcalin, dans l'eau et en présence d'un catalyseur au cuivre puis l'éthérification de l'hydroxy-5 vanilline à l'aide d'un halogénure d'alkyle inférieur, en milieu aqueux et en maintenant le pH entre 6 et 12, éventuellement en présence d'un catalyseur.

Un inconvénient majeur de ce procédé est qu'il y a formation lors de l'hydrolyse de la bromo-5 vanilline d'une quantité relativement importante de vanilline environ 10 à 15 % qui, après la réaction d'éthérification, conduit à l'aldéhyde vératrique (diméthoxy-3,4 benzaldéhyde).

Il est possible de séparer l'aldéhyde vératrique du triméthoxy-3,4,5 benzaldéhyde, par distillation. Cependant, la récupération du triméthoxy-3,4,5 benzaldéhyde à partir du milieu réactionnel est rendue plus compliquée en raison de la présence de l'aldéhyde vératrique.

La présente invention propose un procédé général de préparation de composés aromatiques polyalkoxylés convenant, entre autres, tout à fait bien dans le cas de la préparation du triméthoxy-3,4,5, benzaldéhyde, procédé qui permet d'éviter les inconvénients précités.

L'invention a donc pour objet un procédé de préparation de composés aromatiques polyalkoxylés de formule générale (I) :

$$(R_1-O)_m - Ro - (O-R)_n \qquad (I)$$

dans laquelle :

- m et n sont des nombres entiers supérieurs ou égaux à 1 avec, de préférence, m + n compris entre 2 et 6,
- $R_o$ représente un radical cyclique aromatique ayant au moins 5 atomes dans le cycle, éventuellement substitué, et représentant au moins l'un des radicaux suivants :
  - . un radical carbocyclique aromatique, monocyclique ou polycyclique,
  - . un radical hétérocyclique aromatique, monocyclique ou polycyclique comportant au moins un des hétéroatomes O, N et S,
- R représente un radical hydrocarboné ayant de 1 à 12 atomes de carbone, qui peut être un radical aliphatique acyclique, saturé ou insaturé, linéaire ou ramifié ; un radical cycloaliphatique, saturé ou insaturé, monocyclique ou polycyclique ; un radical cycloaliphatique- ou arylaliphatique, saturé ou insaturé, linéaire ou ramifié,
- $R_1$ est un radical alkyle, linéaire ou ramifié ayant de 1 à 6 atomes de carbone,

procédé caractérisé par le fait que l'on effectue successivement les étapes suivantes :

1°- On fait réagir un composé aromatique porteur d'au moins un atome d'halogène et d'au moins un groupe hydroxyle répondant à la formule générale (II)

$$(HO)_m - Ro - (X)_n \qquad (II)$$

dans laquelle :

- m et n sont des nombres entiers supérieurs ou égaux à 1 avec, de préférence, m + n compris entre 2 et 6,

– X représente un atome d'iode, de brome ou de chlore,

– $R_o$ représente un radical cyclique aromatique ayant au moins 5 atomes dans le cycle, éventuellement substitué, et représentant au moins l'un des radicaux suivants :

. un radical carbocyclique aromatique, monocyclique ou polycyclique,

. un radical hétérocyclique aromatique, monocyclique ou polycyclique comportant au moins un des hétéroatomes O, N et S,

– et un alcoolate de métal alcalin ou alcalino-terreux de formule générale (III) :

$$M^{w+} [ O - R ]_{w}- \qquad (III)$$

dans laquelle :

– M représente un métal alcalin ou alcalino-terreux,

– w représente la valence du métal alcalin ou alcalino-terreux,

– R représente un radical hydrocarboné ayant de 1 à 12 atomes de carbone, qui peut être un radical aliphatique acyclique, saturé ou insaturé, linéaire ou ramifié ; un radical cycloaliphatique, saturé ou insaturé, monocyclique ou polycyclique ; un radical cycloaliphatique- ou arylaliphatique, saturé ou insaturé, linéaire ou ramifié,

en présence d'un catalyseur au cuivre qui est le cuivre et/ou un composé du cuivre et d'une quantité efficace d'un co-catalyseur choisi parmi les carbonates organiques, les carbonates mixtes organo-métalliques, le dioxyde de carbone ou les composés susceptibles de former du dioxyde de carbone,

2° - On réalise la O-alkylation du ou des groupes hydroxyle du produit obtenu précédemment après alkoxylation, par addition directement dans le milieu réactionnel organique d'un agent d'alkylation choisi parmi les halogénures d'alkyle inférieurs, les dialkylsulfates, les dialkylcarbonates.

Dans l'exposé qui suit de la présente invention, on entend par composé aromatique", la notion classique d'aromaticité telle que définie dans la littérature, notamment par Jerry MARCH - Advanced Organic Chemistry, 3ème édition, John Wiley and Sons, 1985 P. 37 et suivantes.

On définit par "composé aromatique polyalkoxylé", un composé aromatique porteur d'au moins deux radicaux alkoxy.

Dans le présent texte, on désigne d'une manière simplifiée par radicaux alkoxy, des radicaux du type R-O- dans lesquels R représente aussi bien un radical aliphatique saturé ou insaturé qu'un radical cycloaliphatique saturé ou insaturé et un radical aliphatique porteur d'un carbocycle saturé, insaturé ou aromatique.

Dans tous les cas, la substitution de l'atome d'halogène par le radical R-O- est dénommé ci-après "réaction d'alkoxylation", quelle que soit la nature du radical R.

Le terme "alcoolate" est utilisé dans le présent texte de manière générique et désigne également les aralkoxydes métalliques.

L'invention s'applique plus particulièrement aux composés aromatiques porteurs d'au moins un atome d'halogène et d'au moins un groupe hydroxyle de formule (II) dans laquelle le radical $R_o$ symbolise :

1° - un radical carbocyclique aromatique, monocyclique ou polycyclique. Par "radical carbocyclique polycyclique", on entend :

. un radical constitué par au moins 2 carbocycles aromatiques et formant entre eux des systèmes ortho ou ortho et péricondensés,

. un radical constitué par au moins 2 carbocycles dont l'un seul d'entre eux est aromatique et formant entre eux des systèmes ortho ou ortho et péricondensés.

2° - un radical hétérocyclique aromatique, monocyclique ou polycyclique. Par "radical hétérocyclique polycyclique", on définit :

. un radical constitué par au moins 2 hétérocycliques contenant au moins un hétéroatome dans chaque cycle dont au moins l'un des deux cycles est aromatique et formant entre eux des systèmes ortho ou ortho et péricondensés,

. un radical constitué par au moins un cycle hydrocarboné et au moins un hétérocycle dont au moins l'un des cycles est aromatique et formant entre eux des systèmes ortho ou ortho et péricondensés.

3° - un radical divalent constitué par un enchaînement de groupements, tels que définis aux paragraphes 1 et/ou 2 liés entre eux :

. par un lien valentiel,

. par un radical alkylène ou alkylidène ayant de 1 à 4 atomes de carbone, de préférence un radical méthylène ou isopropylidène,

. par l'un des groupes suivants :

$-O-$ , $-CO-$ ,

$-S-$ , $-SO-$ , $-SO_2-$ ,

$-N-$ , $-CO-N-$
| |
$R_2$ $R_2$

dans ces formules, $R_2$ représentant un atome d'hydrogène ou un radical alkyle ayant de 1 à 4 atomes de carbone, un radical cyclohexyle ou phényle.

Comme exemples de radicaux listés sous 1° à 3°, on peut citer :

1° - les radicaux phénylène, tolylène, xylylène, naphtylène,

2° - les radicaux pyrrolidinediyle, imidazolidinediyle, pipéridinediyle, furannediyle, pyrrolediyle, thiofènediyle, isoxazolediyle, furazannediyle, isothiazolediyle, imidazolediyle, pyrazolediyle, pyridinediyle, pyridazinediyle, pyrimidinediyle ; les radicaux naphtyridine-1,8 diyle, quinoléïnediyle, indolediyle, benzofurannediyle.

3° - les radicaux biphénylène, méthylène-1,1′ biphénylène, isopropylidène-1,1′ biphénylène, oxy-1,1′ biphénylène, imino-1,1′ biphénylène.

Les composés préférés sont ceux répondant à la formule (II) dans laquelle le radical $R_o$ représente un noyau benzénique.

Comme mentionné précédemment, le radical $R_o$ qui est un radical aromatique porteur d'au moins un groupe hydroxyle et d'au moins un atome d'halogène, peut être également porteur d'un ou plusieurs autres substituants qui peuvent être un autre groupe hydroxyle ou un autre atome d'halogène ou de toute autre nature dans la mesure où ils ne gênent pas la réaction. Généralement, par plusieurs substituants, on définit moins de quatre substituants sur un noyau aromatique.

Comme exemples de substituants donnés à titre illustratif et sans caractère limitatif, on peut mentionner l'un des groupes ou fonctions suivantes :

. un radical de formule $-R_3-OH$ dans laquelle le radical $R_3$ représente un lien valentiel ou un radical hydrocarboné divalent, linéaire ou ramifié, saturé ou insaturé ayant de 1 à 4 atomes de carbone, tel que par exemple, méthylène, éthylène, propylène, isopropylène, isopropylidène,

. un radical alkyle, linéaire ou ramifié, ayant de 1 à 6 atomes de carbone, de préference de 1 à 4 atomes de carbone, tel que méthyle, éthyle, propyle, isopropyle, butyle, isobutyle, sec-butyle, tert-butyle,

. un radical alcényle linéaire ou ramifié ayant de 2 à 6 atomes de carbone, de préférence, de 2 à 4 atomes de carbone, tel que vinyle, allyle,

. un radical alkoxy linéaire ou ramifié ayant de 1 à 6 atomes de carbone, de préférence de 1 à 4 atomes de carbone tel que les radicaux méthoxy, éthoxy, propoxy, isopropoxy, butoxy,

. un groupe -CHO,

. un groupe acyle ayant de 2 à 6 atomes de carbone,

. un radical de formule $-R_3-COOH$, $R_3$ ayant la signification donnée précédemment,

. un radical de formule $-R_3-COOR_4$ dans laquelle $R_3$ a la signification donnée précédemment et $R_4$ représente un radical alkyle, linéaire ou ramifié, ayant de 1 à 6 atomes de carbone,

. un radical de formule $-R_3-NH_2$ avec un groupe $NH_2$ N-protégé, $R_3$ ayant la signification donnée précédemment,

. un radical de formule $-R_3-N(R'_4)_2$ dans laquelle $R_3$ a la signification donnée précédemment et les radicaux $R'_4$ identiques ou différents représentent un atome d'hydrogène ou un radical alkyle, linéaire ou ramifié, ayant de 1 à 6 atomes de carbone,

. un radical de formule $-R3-CO-N(R'_4)_2$, $R_3$ et $R'_4$ ayant la signification donnée précédemment,

. un radical de formule $-R_3-z$ dans laquelle $R_3$ a la signification donnée précédemment et z symbolise un atome d'halogène X, tel que précédemment défini, un atome de fluor ou un groupe $CF_3$.

S'il y a présence de substituants sur le cycle aromatique, il y a lieu de veiller que celui-ci n'interfère pas au niveau du produit désiré.

De même, lors de la présence d'une fonction amine primaire, il peut être nécessaire de la N-protéger par un groupe protecteur, par exemple acyle, et plus spécialement acétyle.

Si le cycle est porteur d'un groupe du type $-R_3-COOR_4$, le groupe $R_4$ sera échangé par le groupe R provenant de l'alcoolate de métal alcalin ou alcalino-terreux, si $R_4$ et R sont des groupes alkyles de nature différente.
Si le cycle est porteur d'une fonction acide du type -$R_3$-COOH, le groupe sera salifié et entraînera donc une consommation supplémentaire de l'alcoolate utilisé.
Lorsque le cycle aromatique est porteur d'une chaîne aliphatique latérale avec présence d'un atome d'halogène X, celui-ci peut être substitué également par le groupe R-O- provenant de l'alcoolate de métal alcalin ou alcalino-terreux et il y aura donc lieu d'en tenir compte pour la détermination des quantités de réactifs à mettre en oeuvre.
Comme exemples de substituants cités à titre préférentiel et sans caractère limitatif, on peut mentionner :

- un ou plusieurs radicaux alkyle ayant de 1 à 4 atomes de carbone,
- un ou plusieurs radicaux alkoxy ayant de 1 à 4 atomes de carbone,
- un ou plusieurs radicaux hydroxyle.

A titre d'exemples de composés aromatiques porteurs d'au moins un atome d'halogène et d'au moins un groupe hydroxyle répondant à la formule générale (II), on peut citer plus particulièrement :

- le bromo-2 phénol
- le bromo-3 phénol
- le bromo-4 phénol
- le bromo-1 naphtol-2
- le bromo-6 naphtol-2
- le bromo-2 méthyl-4 phénol
- le bromo-4 diméthyl-2,6 phénol
- le bromo-4 diméthyl-3,5 phénol
- le dibromo-2,6 méthyl-4 phénol
- le bromo-2-p-crésol
- le bromo-2 chloro-4 phénol
- le bromo-4 chloro-2 phénol
- le bromo-4 chloro-6-o-crésol
- les bromofluorophénols
- les bromo bis-phénols
- les bromo isopropylidène-4,4' bis-phénols

L'alcoolate de métal alcalin ou alcalino-terreux qui intervient dans le procédé de l'invention répond à la formule (III) dans laquelle w est égal à 1 ou 2 et R représente :

1° - un radical alkyle, alcényle, alcadiényle, alcynyle linéaire ou ramifié ayant, de préférence, moins de 6 atomes de carbone,
2° - un radical cycloalkyle ou cycloalcényle ayant, de préférence, de 5 à 7 atomes de carbone et l'on peut citer en particulier le radical cyclohexyle,
3° - un radical aliphatique acyclique, saturé ou insaturé, linéaire ou ramifié tel que précisé sous 1°, porteur d'un substituant cyclique. Par cycle, on entend un cycle carbocyclique saturé, insaturé ou aromatique. Comme exemple, on peut mentionner le radical benzyle.

Parmi les alcoolates précités, on met en oeuvre de préférence dans le procédé de l'invention, les alcoolates de métaux alcalins et plus particulièrement les alcoolates de sodium ou de potassium des alcanols primaires ou secondaires ayant 1 à 4 atomes de carbone conviennent particulièrement bien.
Les plus fréquemment utilisés sont le méthylate de sodium et l'éthylate de sodium.
Un mode de mise en oeuvre préférentiel de l'invention réside dans un procédé de préparation d'un composé aromatique polyalkoxylé entrant dans la définition de la formule (I) et répondant plus particulièrement à la formule générale (Ia) :

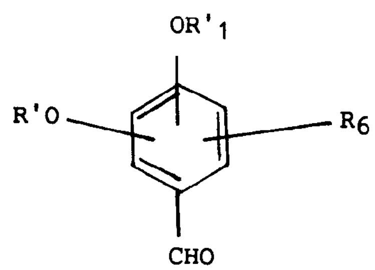

(Ia)

dans laquelle:

– R′, $R'_1$, $R_6$ identiques ou différents représentent un radical alkyle, linéaire ou ramifié, ayant de 1 à 4 atomes de carbone,

– $R_6$ pouvant représenter également un atome d'hydrogène, un radical alkoxy, linéaire ou ramifié ayant de 1 à 4 atomes de carbone.

Le procédé selon l'invention, de préparation d'un composé aromatique polyalkoxylé répondant à la formule générale (Ia) comporte les étapes suivantes:

1° - On fait réagir un composé aromatique entrant dans la définition générale de la formule (II) qui est un halogéno hydroxy benzaldéhyde répondant à la formule générale (IIa) :

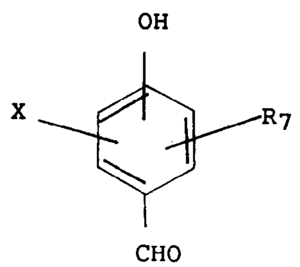

(IIa)

dans laquelle:

– X représente un atome d'iode, de brome ou de chlore,

– $R_7$ représente un atome d'hydrogène, un atome d'iode, de brome, un atome de chlore, un radical alkyle ayant de 1 à 4 atomes de carbone, un radical alkoxy ayant de 1 à 4 atomes de carbone, un radical hydroxyle,

dans ladite formule (IIa), le radical hydroxyle pouvant être en position ortho-, méta- ou para- de la fonction aldéhyde,

avec un alcoolate de métal alcalin ou alcalino-terreux ayant de 1 à 4 atomes de carbone, en présence d'un catalyseur au cuivre qui est le cuivre et/ou un composé du cuivre et d'une quantité efficace d'un co-catalyseur choisi parmi les carbonates organiques, les carbonates mixtes organométalliques, le dioxyde de carbone ou les composés susceptibles de former du dioxyde de carbone,

2° - On réalise la O-alkylation du ou des groupes hydroxyle du produit obtenu précédemment après alkoxylation, par addition directement dans le milieu réactionnel organique d'un agent d'alkylation apportant le reste alkyle $R'_1$ ayant de 1 à 4 atomes de carbone.

Comme exemples de substrats mis en oeuvre préférentiellement, on peut citer plus particulièrement les composés répondant à la formule générale (IIa) dans laquelle :

. Groupe I

– le radical OH est en position para par rapport à la fonction CHO,
– l'atome d'halogène X est en position ortho par rapport au radical OH.
– le radical $R_7$ est en position ortho par rapport au radical OH

. Groupe II

– le radical OH est en position ortho par rapport à la fonction CHO.
– l'atome d'halogène X est en position para par rapport au radical OH,
– le radical $R_7$ est en position ortho par rapport au radical OH,

. Groupe III

– le radical OH est en position ortho par rapport à la fonction CHO,
– l'atome d'halogène X est en position ortho par rapport au radical OH,
– le radical $R_7$ est en position para par rapport au radical OH,

. Groupe IV

– le radical OH est en position méta par rapport à la fonction CHO,
– l'atome d'halogène X est en position ortho par rapport au radical OH,

– le radical $R_7$ est en position para par rapport au radical OH.

. Groupe V

– le radical OH est en position méta par rapport à la fonction CHO,
– l'atome d'halogène X est en position para par rapport au radical OH,
– le radical $R_7$ est en position ortho par rapport au radical OH.

Les composés de formule (IIa) mis en oeuvre préférentiellement, répondent plus précisément aux formules suivantes:

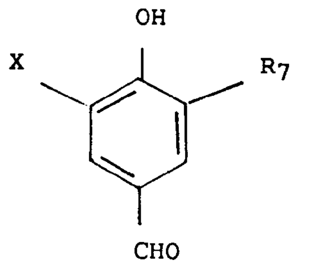

$(IIa_1)$

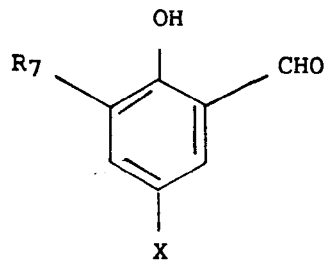

$(IIa_2)$

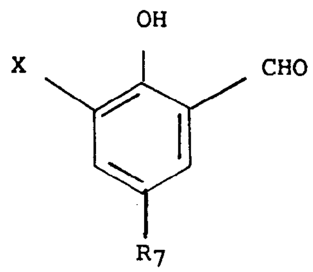

$(IIa_3)$

dans lesquelles:

– X et $R_7$ ont les significations données précédemment.

Dans le procédé de l'invention, on met tout préférentiellement en oeuvre, un halogéno hydroxy benzaldéhyde répondant à la formule générale ($IIa_1$) dans laquelle X est un atome de brome et $R_7$ symbolise un radical méthoxy ou éthoxy.

A titre d'exemples d'halogéno hydroxy benzaldéhydes répondant à la formule (IIa) qui servent de substrats de départ dans le présent procédé, on peut citer plus précisément :

– le bromo-3 hydroxy-4 benzaldéhyde,
– le iodo-3 hydroxy-4 benzaldéhyde
– le dibromo-3,5 hydroxy-4 benzaldéhyde
– le diiodo-3,5 hydroxy-4 benzaldéhyde
– le bromo-5 méthoxy-3 hydroxy-4 benzaldéhyde
– le iodo-5 méthoxy-3 hydroxy-4 benzaldéhyde
– le bromo-5 éthoxy-3 hydroxy-4 benzaldéhyde
– le iodo-5 éthoxy-3 hydroxy-4 benzaldéhyde
– le bromo-3 dihydroxy-4,5 benzaldéhyde
– le iodo-3 dihydroxy-4,5 benzaldéhyde
– le bromo-3 dihydroxy-2,5 benzaldéhyde

– le iodo-3 dihydroxy-2,5 benzaldéhyde
– le bromo-2 hydroxy-4 benzaldéhyde
– le iodo-2 hydroxy-4 benzaldéhyde
– le bromo-4 hydroxy-3 benzaldéhyde
– le iodo-4 hydroxy-3 benzaldéhyde
– le bromo-3 hydroxy-2 benzaldéhyde
– le iodo-3 hydroxy-2 benzaldéhyde
– le bromo-5 hydroxy-2 benzaldéhyde
– le iodo-5 hydroxy-2 benzaldéhyde

Dans un mode préférentiel de mise en oeuvre du procédé de l'invention pour la préparation de composés aromatiques polyalkoxylés de formule (Ia), l'alcoolate de métal alcalin ou alcalino-terreux de formule (III) est de préférence un alcoolate de sodium ou potassium d'un alcanol primaire ou secondaire ayant de 1 à 4 atomes de carbone et, de préférence, le méthylate ou l'éthylate de sodium.

Conformément au procédé de l'invention, on fait réagir le composé aromatique porteur d'au moins un atome d'halogène et d'au moins un groupe hydroxyle répondant à la formule (II) avec un alcoolate de métal alcalin ou alcalino-terreux de formule (III), en présence d'un catalyseur au cuivre et d'un co-catalyseur choisi parmi les carbonates organiques, les carbonates mixtes organo-métalliques, le dioxyde de carbone ou les composés susceptibles de former du dioxyde de carbone.

Les composés du cuivre servant de catalyseurs sont connus. Ce sont d'une manière générale tous les composés organiques ou inorganiques du cuivre I ou du cuivre II.

Le cuivre métal peut être utilisé, mais son action est plus lente car il faut préalablement qu'il se transforme en partie en cuivre I ou cuivre II.

A titre non limitatif, on peut citer comme composés du cuivre, le chlorure cuivreux, le chlorure cuivrique, le carbonate de cuivre II basique, le nitrate cuivreux, le nitrate cuivrique, le sulfate cuivrique, l'acétate cuivrique, le trifluorométhylsulfonate cuivrique, l'hydroxyde cuivrique, le picolinate de cuivre II, le méthylate de cuivre 1, le méthylate de cuivre II, le chélate de cuivre II de la 8-quinoléine, les composés de cuivre de formule $ClCuOCH_3$, $Cu_2(OCH_3)_2(acac)_2$.

Les carbonates organiques et les carbonates mixtes organométalliques utilisés dans l'invention sont plus particulièrement les carbonates de formule générale (IV)

$$[R_8 - O - CO - O -]p - R_9$$

dans laquelle:

– $R_8$ représente :
. un radical alkyle, linéaire ou ramifié, ayant 1 à 6 atomes de carbone,
. un radical cycloalkyle ayant 5 à 6 atomes de carbone,
. un radical cycloalkyle, ayant 5 ou 6 atomes de carbone substitué par un ou deux radicaux alkyle ayant 1 à 12 atomes de carbone,
. un radical aryle ayant de 6 à 12 atomes de carbone,
. un radical aryle substitué par 1 ou 2 radicaux alkyle ayant 1 à 12 atomes de carbone ;
. un radical $R_{10}$ - O - CO - dans lequel $R_{10}$ représente un radical alkyle, linéaire ou ramifié ayant 1 à 6 atomes de carbone, un radical aryle ayant de 6 à 12 atomes de carbone, un radical cycloalkyle ayant 5 ou 6 atomes de carbone ;

– $R_9$ représente :
. un radical alkyle, linéaire ou ramifié, ayant 1 à 6 atomes de carbone,
. un radical cycloalkyle ayant 5 ou 6 atomes de carbone,
. un radical cycloalkyle ayant 5 ou 6 atomes de carbone substitué par un ou deux radicaux alkyle ayant 1 à 12 atomes de carbone,
. un radical aryle ayant 6 à 12 atomes de carbone,
. un radical aryle substitué par 1 ou 2 radicaux alkyle ayant 1 à 12 atomes de carbone,
. un métal alcalin ou alcalino-terreux, de préférence le sodium ou le potassium.

– p = 1 ou p = 2 lorsque $R_9$ représente un métal alcalino-terreux ;
– $R_8$ et $R_9$ peuvent former ensemble un radical alkylène ayant 2 à 6 atomes de carbone.

De préférence, les carbonates organiques et les carbonates mixtes organo-métalliques sont les carbonates de formule générale (IVa) :

$$R_8 - O - CO - O - R_9 \qquad (IV_a)$$

dans laquelle:

– $R_8$ représente
. un radical alkyle, linéaire ou ramifié, ayant 1 à 6 atomes de carbone,
. un radical cycloalkyle ayant 5 à 6 atomes de carbone,

. un radical cycloalkyle, ayant 5 ou 6 atomes de carbone substitué par un ou deux radicaux alkyle ayant 1 à 4 atomes de carbone,

. un radical phényle,

. un radical phényle substitué par 1 ou 2 radicaux alkyle ayant 1 à 4 atomes de carbone,

. un radical $R_{10}$ - O - CO - dans lequel $R_{10}$ représente un radical alkyle, linéaire ou ramifié ayant 1 à 6 atomes de carbone, un radical phényle, un radical cycloalkyle ayant 5 ou 6 atomes de carbone

– $R_9$ représente:

. un radical alkyle, linéaire ou ramifié, ayant 1 à 6 atomes de carbone,

. un radical cycloalkyle ayant 5 ou 6 atomes de carbone, un radical cycloalkyle ayant 5 ou 6 atomes de carbone substitué par un ou deux radicaux alkyle ayant 1 à 4 atomes de carbone,

. un radical phényle,

. un radical phényle substitué par un ou deux radicaux alkyle ayant 1 à 4 atomes de carbone,

. un atome de sodium ou de potassium,

– $R_8$ et $R_9$ peuvent former ensemble un radical alkylène ayant 2 à 6 atomes de carbone.

Comme exemples de carbonates organiques ou organo-métalliques,on peut citer : le carbonate de ditertiobutyle, le carbonate de diéthyle, le carbonate de diméthyle, le carbonate d'éthylène, le carbonate de propylène, le carbonate de phényle et de tertiobutyle, le carbonate de tertiobutyle et de sodium, le dicarbonate de ditertiobutyle.

En ce qui concerne le dioxyde de carbone, il peut être mis en oeuvre en solution dans le milieu réactionnel, par circulation dans celui-ci.

Comme exemples de composés susceptibles de former du dioxyde de carbone dans le milieu réactionnel, on peut citer :

– les $\alpha$(ou $\beta$)cétoesters et les $\alpha$(ou $\beta$)cétoacides ;

– les carbonates d'amine, les urées et les carbodiimides ;

– les acides dicarboxyliques comme l'acide malonique, les acides $\alpha$-cyano ou $\alpha$-nitro-carboxyliques ;

– les acides carboxyliques $\beta\gamma$-insaturés.

Conformément au procédé de l'invention, on soumet le composé aromatique porteur d'au moins un atome d'halogène et d'au moins un groupe hydroxyle répondant à la formule (II), à une réaction d'alkoxylation en faisant réagir ledit composé avec l'alcoolate de métal alcalin ou alcalino-terreux, en présence d'un catalyseur au cuivre et d'un co-catalyseur de type $CO_2$ ou carbonate, puis à soumettre le composé aromatique ainsi obtenu porteur d'au moins un groupe alkoxy et d'au moins un groupe hydroxyle, à une réaction de O-alkylation en le faisant réagir avec un agent d'alkylation.

Selon la présente invention, on prépare, par exemple, un dialkoxy-3,4 et un trialkoxy-3,4,5 benzaldéhyde à partir respectivement d'un halogéno-3 hydroxy-4 benzaldéhyde et d'un halogéno-5 hydroxy-4 alkoxy-3 benzaldéhyde de formule (IIa) en soumettant les substrats de départ, d'abord à une réaction d'alkoxylation puis à une réaction de O-alkylation.

Dans le cas où le composé de formule (II) présente un ou plus d'un atome d'halogène et un ou plus d'un groupe hydroxyle, on fera respectivement une mono- ou poly-alkoxylation dans la première étape ou une mono- ou poly-O-alkylation dans la deuxième étape, la seule condition étant uniquement d'adapter les quantités de réactifs en fonction du nombre de groupes à substituer.

La première étape du procédé de l'invention réside donc dans l'alkoxylation du composé aromatique porteur d'au moins un atome d'halogène et d'au moins un groupe hydroxyle répondant à la formule (II).

Dans le cas où ledit composé répond plus particulièrement à la formule générale (IIa) c'est-à-dire lorsqu'il est également porteur d'une fonction aldéhyde, on le fait réagir selon l'invention avec un alcoolate de métal alcalin ou alcalino-terreux, en présence d'un catalyseur au cuivre et d'un co-catalyseur tel que précité ce qui permet ainsi de conduire la réaction d'alkoxylation sans qu'il soit nécessaire de protéger la fonction aldéhyde, sous forme d'acétal. Un tel procédé fait l'objet des demandes de brevets FR 89/13370 et EP 90402786.9 au nom de la demanderesse.

Selon le procédé de l'invention, l'alkoxylation du composé aromatique porteur d'au moins un atome d'halogène et d'au moins un groupe hydroxyle répondant à la formule (II) est conduite en milieu organique constitué, le plus souvent, par l'alcanol correspondant à l'alcoolate de métal alcalin ou alcalino-terreux utilisé.

On choisit, préférentiellement comme solvant, le méthanol ou l'éthanol.

Lorsque l'alcoolate de métal alcalin ou alcalino-terreux présente une condensation en carbone supérieure à 4 atomes de carbone, il est souhaitable de faire appel à un solvant inerte vis-à-vis de la réaction et de choisir, de préférence, un solvant aprotique polaire.

Comme exemples de solvants aprotiques polaires convenant à la mise en oeuvre du procédé de l'invention, on peut mentionner, plus particulièrement les éthers et, plus précisément, les éthers diméthyliques dérivant de l'oxyde d'éthylène ou de l'oxyde de propylène tels que le diméthyléther de l'éthylèneglycol (ou diméthoxy-1,2

éthane), le diméthyléther du diéthylèneglycol (ou diméthoxy-1,5 oxa-3 pentane), le diméthoxy-1,8 dioxa-3,6 octane, le diméthoxy-1,11 trioxa-3,6,9 undécane, le diméthoxy-1,2 méthyl-1 éthane, le diméthoxy-1,5 diméthyl-1,4 oxa-3 pentane, le diméthoxy-1,7 diméthyl-1,4 dioxa-3,6 octane.

On peut également utiliser plusieurs solvants.

Parmi tous les solvants précités, le diméthyléther de l'éthylèneglycol et le diméthyléther du diéthylèneglycol sont préférés.

La concentration du composé de formule (II) exprimée en poids dudit composé (II) par rapport au poids total composé (II) + solvant est généralement de 3 à 40 % et, de préférence, de 10 à 30 %.

La quantité d'alcoolate de métal alcalin ou alcalino-terreux utilisée est égale ou supérieure à la quantité stoechiométrique nécessaire, d'une part pour transformer le ou les atomes d'halogène en groupements alkoxy et d'autre part pour transformer le composé de formule (II) en phénate de métal alcalin ou alcalino-terreux dans le cas où ledit composé présente un ou plusieurs radicaux hydroxyle. On effectue donc la salification du ou des groupements OH.

Généralement, l'alcoolate de métal alcalin ou alcalino-terreux est mis en oeuvre en une quantité correspondant à la quantité stoechiométrique nécessaire pour salifier les groupes hydroxyle plus une quantité égale de 1 fois à 5 fois, et de préférence de 1 à 3 fois, la quantité stoechiométrique nécessaire pour transformer le ou les atomes d'halogène en groupements alkoxy.

La concentration de l'alcoolate de métal alcalin ou alcalinoterreux est avantageusement supérieure à 1 mole/litre et, de préférence comprise entre 1 et 5 moles/litre. Il est à noter que la borne supérieure ne présente aucun caractère critique.

De manière pratique, l'alcoolate de métal alcalin ou alcalinoterreux est formé in situ, en faisant réagir un excès d'alcanol avec le métal alcalin ou alcalino-terreux choisi.

La quantité de catalyseur au cuivre utilisée dans le procédé de l'invention peut varier très largement.

Habituellement, le rapport molaire catalyseur au cuivre/composé de formule (II) est de 1 à 50 % et, de préférence, de 2 % à 20 %.

La quantité de co-catalyseur mis en oeuvre dans le procédé de l'invention est telle que l'on ait un rapport molaire co-catalyseur/catalyseur au cuivre de 1 à 10 et, de préférence, de 1 à 5.

La température de la réaction d'alkoxylation est généralement comprise entre 60°C et 220°C, et, de préférence entre 100°C et 180°C.

La pression n'est pas un paramètre critique en soi, mais pour atteindre les températures indiquées précédemment et ne pas perdre de solvant, le procédé est habituellement réalisé sous pression autogène.

Généralement, cette pression autogène du mélange réactionnel est inférieure ou égale à 5 MPa (50 bars).

La durée de la réaction d'alkoxylation peut varier largement entre 1 et 10 heures, de préférence entre 2 et 5 heures.

Dans la deuxième étape du procédé de l'invention, on conduit la réaction de O-alkylation du composé aromatique précédemment obtenu qui est porteur d'au moins un groupe alkoxy et d'au moins un groupe hydroxyle.

A cet effet, on le met en contact avec un agent d'alkylation. On peut faire appel aux alkylsulfates de formule $R_1$-O-$SO_2$-O-$R_1$ ou aux alkylcarbonates de formule $R_1$-O-CO-O-$R_1$, dans lesdites formules, $R_1$ représente un radical alkyle, linéaire ou ramifié, ayant de 1 à 6 atomes de carbone.

Parmi les agents d'alkylation précités, le diméthylsulfate ou le diméthylcarbonate sont préférés.

Toutefois, on préfère selon l'invention choisir, à titre d'agent d'alkylation, un halogénure d'alkyle inférieur répondant à la formule générale (V) :

$$R_1\text{-}X \qquad (V)$$

dans ladite formule, X représente un atome de brome, de chlore ou d'iode et $R_1$ représente un radical alkyle linéaire ou ramifié ayant de 1 à 6 atomes de carbone.

Parmi les halogénures de formule (V), on préfère mettre en oeuvre ceux répondant à la formule (V) dans laquelle X est un atome de chlore et $R'_1$ un radical alkyle linéaire ou ramifié ayant de 1 à 4 atomes de carbone.

Le plus souvent, on utilise un halogénure de méthyle ou un halogénure d'éthyle.

Parmi les halogénures, les chlorures, bromures et iodures sont généralement mis en oeuvre et plus spécifiquement encore le chlorure de méthyle, le chloroéthane, le bromure de méthyle et le bromoéthane.

En raison de leur plus faible coût, on préfère utiliser le chlorure de méthyle et le chloroéthane.

Une variante d'exécution de l'invention consiste à ajouter dans le milieu réactionnel, de préférence avant l'addition de l'agent d'alkylation, un sel sous forme d'iodure, afin d'avoir une cinétique plus élevée. Comme sels, on peut citer les iodures de métal alcalin, notamment de sodium, de potassium ou de lithium.

Généralement, la réaction de O-alkylation est enchaînée directement après la réaction d'alkoxylation, en introduisant l'agent d'alkylation à la température requise. Toutefois, dans certains cas, il peut être plus avantageux de maintenir le pH, à un pH compris entre 6 et 12 pendant la durée de la réaction.

Lorsque l'on opère à un pH inférieur à 6, on observe une diminution de la réaction de O-alkylation ainsi

qu'un ralentissement de cette reaction.

D'une manière préférentielle, le pH du milieu est maintenu entre 9 et 11.

La régulation du pH peut être si nécessaire assurée par addition continue d'une base, de préférence, une solution aqueuse d'un hydroxyde de métal alcalin. Tous les hydroxydes de métal alcalin peuvent être utilisés. Cependant pour des considérations économiques, la soude est préférée.

Pour ce qui est de la quantité des réactifs à mettre en oeuvre dans la deuxième étape du procédé de l'invention, on définit ci-après les quantités préférées.

La quantité d'agent d'alkylation engagée est fonction du nombre de groupes hydroxyle présents sur le noyau aromatique du produit obtenu précédemment, après alkoxylation. Elle est de préférence au moins égale à quantité stoechiométrique jusqu'à un excès pouvant atteindre 200 %. Elle est, de préférence, égale à la quantité stoechiométrique.

A titre d'exemples, on précisera que le rapport molaire halogénure d'alkyle/alkoxy hydroxy benzaldéhyde varie de 1,0 à 3,0 et, de préférence, voisin de 1,0 et que le rapport molaire halogénure d'alkyle/alkoxy dihydroxy benzaldéhyde varie de 2,0 à 6,0 et, de préférence, voisin de 2,0.

La quantité de base est déterminée de telle sorte que le pH soit maintenu dans l'intervalle précité.

La quantité de sel sous forme d'iodure mis en oeuvre selon une autre variante préférée de l'invention peut également varier largement. Habituellement, le rapport molaire entre le sel sous forme d'iodure et l'agent d'alkylation varie entre 0,05 et 0,20 et se situe de préférence, aux environs de 0,10.

En ce qui concerne les conditions réactionnelles, la température de la réaction de O-alkylation n'est pas critique ; elle a une influence sur la cinétique de la réaction. Généralement, on réalise la réaction de O-alkylation entre 80°C et 200°C. De préférence, on opère à une température entre 100°C et 160°C.

La pression n'est pas critique. Elle a une incidence sur la cinétique de la réaction. Elle varie habituellement entre la pression atmosphérique et 50 bars et se situe, de préférence entre la pression atmosphérique et 20 bars.

La pression est habituellement créée par le solvant réactionnel et l'agent d'alkylation servant à la O-alkylation lorsqu'ils sont gazeux dans les conditions réactionnelles.

La durée de la réaction de O-alkylation est fonction notamment de la température réactionnelle. Elle varie le plus souvent entre 1 heure et 8 heures. Généralement, une durée de 1 à 4 heures est suffisante.

Selon un mode de réalisation pratique de l'invention, on peut mélanger tous les réactifs de la première étape à savoir le composé aromatique de formule (II), le catalyseur au cuivre et le co-catalyseur de type $CO_2$ ou carbonate et après chauffage pendant la durée nécessaire à la réaction d'alkoxylation, ajouter dans le milieu réactionnel, l'agent d'alkylation éventuellement une base.

Un mode préféré de mise en pratique de l'invention consiste d'abord à préparer une solution de l'alcoolate de métal alcalin dans l'alcanol correspondant à raison de, par exemple, 20 à 50 % en poids.

Dans l'appareillage, on charge sous atmosphère de gaz inerte, de préférence, l'azote, le composé aromatique de formule (II), le catalyseur au cuivre puis le co-catalyseur de type $CO_2$ ou carbonate, puis l'on introduit la solution de l'alcoolate de métal alcalin. Dans le cas du dioxyde de carbone, on envoie donc un courant gazeux dans le milieu réactionnel.

On porte ensuite le milieu réactionnel à la température choisie entre 60°C et 220°C, de préférence, entre 100°C et 180°C, pendant la durée pré-définie.

On introduit ensuite l'agent d'alkylation, de préférence, l'halogénure d'alkyle qui peut être liquide ou gazeux et on injecte ou coule en parallèle la solution d'hydroxyde de métal alcalin, si celle-ci est nécessaire pour ajuster le pH dans la zone précitée.

On maintient le chauffage entre 80°C et 200°C, de préférence entre 100°C et 160°C, pendant la durée nécessaire à la réaction de O-alkylation.

En fin de réaction, on sépare le composé aromatique polyalkoxylé de formule (I) obtenu, selon les techniques classiques de séparation, soit par distillation, soit par extraction par un solvant approprié, par exemple, le toluène dans le cas du triméthoxy-3,4,5 benzaldéhyde.

Il est possible d'éliminer le catalyseur au cuivre du milieu réactionnel selon les traitements classiques, notamment par traitement acide.

Un des avantages du procédé de l'invention est qu'il permet d'effectuer à la suite et dans le même réacteur, la réaction d'alkoxylation et de O-alkylation.

Dans le cas de la préparation du triméthoxy-3,4,5 benzaldéhyde, on effectue sa préparation à partir de bromo-5 hydroxy-4 méthoxy-3 benzaldéhyde, en milieu organique essentiellement homogène, sans séparation du produit intermédiaire alkoxylé, ce qui est très avantageux d'un point de vue industriel.

Par rapport au procédé décrit dans FR-A 2 609 984, la séparation du triméthoxy-3,4,5 benzaldéhyde est plus simple, car il n'y a pas de formation d'aldéhyde vératrique qu'il faut ensuite séparer.

Le procédé de l'invention est donc particulièrement bien adapté à la préparation des alkoxybenzaldéhydes

suivants :

- le triméthoxy-3,4,5 benzaldéhyde
- le diméthoxy-3,4 benzaldéhyde
- le diméthoxy-2,5 benzaldéhyde
- le diéthoxy-3,4 benzaldéhyde
- l'éthoxy-3 méthoxy-4 benzaldéhyde

Le triméthoxy-3,4,5 benzaldéhyde qui peut être préparé selon le procédé de l'invention sert notamment pour la préparation de produits pharmaceutiques.

Les exemples qui suivent illustrent l'invention sans toutefois la limiter.

Dans les exemples, les abréviations suivantes signifient:

BHMB : bromo-5 hydroxy-4 méthoxy-3 benzaldéhyde
TMBA : triméthoxy-3,4,5 benzaldéhyde
syringaldéhyde : hydroxy-4 diméthoxy-3,5 benzaldéhyde
BHB : bromo-5 hydroxy-2 benzaldéhyde
DMBA : diméthoxy-2,5 benzaldéhyde

$$TT_{\text{BHMB (ou BHB)}} = \frac{\text{Nombre de moles de BHMB (ou BHB) transformées}}{\text{Nombre de moles de BHMB (ou BHB) introduites}} \%$$

$$RT_{\text{TMBA (ou DMBA)}} = \frac{\text{Nombre de moles de TMBA (ou DMBA) formées}}{\text{Nombre de moles de BHMB (ou BHB) transformées}} \%$$

$$RT_{\text{syringaldéhyde}} = \frac{\text{Nombre de moles de syringaldéhyde formées}}{\text{Nombre de moles de BHMB transformées}} \%$$

## EXEMPLE 1

Dans un réacteur de 3,9 litres en acier inoxydable, muni d'un système de chauffage et d'une agitation, on charge sous atmosphère d'azote :

- 173,2 g (0,750 mole) de bromo-5 hydroxy-4 méthoxy-3 benzaldéhyde (BHMB)
- 162 g de méthylate de sodium
- 1785 $cm^3$ (1428 g) de méthanol
- 8,3 g de carbonate de cuivre II basique de formule $CuCO_3,Cu(OH)_2$
- 16 g de dioxyde de carbone

Le dioxyde de carbone est apporté sous forme gazeuse, par bullage dans le mélange réactionnel.

On chauffe sous agitation, pendant 4 heures, à 125°C, sous pression autogène, afin de réaliser la réaction de méthoxylation.

On refroidit le mélange réactionnel à 120°C puis l'on charge 150 g de chlorure de méthyle.

On maintient le mélange réactionnel à 120°C pendant 3 heures.

On refroidit à température ambiante.

On sépare par filtration la partie insoluble.

On dose par chromatographie liquide haute performance, le BHMB n'ayant pas réagi et le triméthoxy-3,4,5 benzaldéhyde obtenu.

Les résultats obtenus sont les suivants:

- taux de transformation du BHMB ($TT_{BHMB}$ %) = 100 %
- rendement en TMBA ($RT_{TMBA}$ %) = 92 %

## EXEMPLE 2

On répète l'exemple 1 avec les mêmes charges, mais en modifiant les conditions opératoires.

On réalise la réaction de méthoxylation, en chauffant sous agitation pendant 4 heures, à 125°C, sous pression autogène.

On refroidit le mélange réactionnel à 100°C puis l'on charge 150 g de chlorure de méthyle.

On maintient le mélange réactionnel à 100°C pendant 3 heures.

On refroidit à température ambiante.

On sépare par filtration la partie insoluble.

On dose par chromatographie liquide haute performance, le BHMB n'ayant pas réagi, le TMBA et le syringaldéhyde obtenus.

Les résultats obtenus sont les suivants :

- taux de transformation du BHMB ($TT_{BHMB}$ %) = 100 %
- rendement en syringaldéhyde ($RT_{syringaldéhyde}$ %) = 18,5 %
- rendement en TMBA ($RT_{TMBA}$ %) = 79 %

EXEMPLE 3

On répète l'exemple 1 avec les mêmes charges, mais en modifiant les conditions opératoires.

On réalise la réaction de méthoxylation, en chauffant sous agitation pendant 3 heures, à 135°C, sous pression autogène.

On refroidit le mélange réactionnel à 120°C puis l'on charge 150 g de chlorure de méthyle.

On maintient le mélange réactionnel à 120°C pendant 1 heure.

On refroidit à tempéature ambiante.

On sépare par filtration la partie insoluble.

On dose par chromatographie liquide haute performance, le BHMB n'ayant pas réagi et le TMBA obtenu.

Les résultats obtenus sont les suivants.

- taux de transformation du BHMB ($TT_{BHMB}$ %) = 100 %
- rendement en TMBA ($RT_{TMBA}$ %) = 97 %

EXEMPLE 4

Dans un réacteur de 3,9 litres en acier inoxydable, muni d'un système de chauffage et d'une agitation, on charge sous atmosphère d'azote :

- 150 g (0,750 mole) de bromo-5 hydroxy-2 benzaldéhyde (BHMB)
- 162 g de méthylate de sodium
- 1785 $cm^3$ (1428 g) de méthanol
- 8,3 g de carbonate de cuivre II basique de formule $CuCO_3,Cu(OH)_2$
- 16 g de dioxyde de carbone

Le dioxyde de carbone est apporté sous forme gazeuse, par bullage dans le mélange réactionnel.

On chauffe sous agitation, pendant 5 heures, à 125°C, sous pression autogène, afin de réaliser la réaction de méthoxylation.

On refroidit le mélange réactionnel à 120°C puis l'on charge 150 g de chlorure de méthyle.

On maintient le mélange réactionnel à 120°C pendant 3 heures.

On refroidit à température ambiante.

On sépare par filtration la partie insoluble.

On dose par chromatographie liquide haute performance, le BHB n'ayant pas réagi et le diméthoxy-2,5 benzaldéhyde obtenu.

Les résultats obtenus sont les suivants:

- taux de transformation du BHB ($TT_{BHB}$ %) = 75 %
- rendement en DMBA ($RT_{DMBA}$ %) = 81 %

EXEMPLE 5

Dans un réacteur téfloné de 40 $cm^3$, muni d'un système de chauffage et d'une agitation, on charge :

- 2,31 g (10 mmol) de bromo-5-hydroxy-4-méthoxy-3 benzaldéhyde (BHMB)
- 25 g d'une solution méthanolique contenant 2,16 g (40 mmol) de méthylate de sodium
- 0,110 g (0,5 mmol) de carbonate de cuivre II basique de formule $CuCO_3,Cu(OH)_2$
- 15 $cm^3$ de méthanol

On chauffe sous agitation pendant 3 heures à 125°C, puis on introduit 1,80 g (20 mmol) de diméthylcarbonate. On porte à 160°C, endant 4 heures et sous pression autogène.

On refroidit à température ambiante, on dilue avec de l'eau distillée. On ajuste le pH du mélange réactionnel

à 4 à l'aide d'acide sulfurique concentré. On filtre la partie insoluble.

On dose par chromatographie en phase liquide.

Les résultats obtenus sont les suivants :

- taux de transformation du BHMB ($TT_{BHMB}$ %) = 100 %
- rendement en syringaldéhyde ($RT_{syringaldéhyde}$ %) = 19 %
- rendement en TMBA ($RT_{TMBA}$ %) = 81 %

EXEMPLE 6

On répète l'exemple 5 avec les mêmes charges et les mêmes conditions opératoires, mais en réduisant la quantité de méthanol à 8 $cm^3$ et en rajoutant 0,30 g (2 mmol) d'iodure de sodium, à l'étape de méthylation, mais avant l'addition du diméthylcarbonate.

Les résultats obtenus sont les suivants :

- taux de transformation du BHMB ($TT_{BHMB}$ %) = 100 %
- rendement en syringaldéhyde ($RT_{syringaldéhyde}$ %) = 14 %
- rendement en TMBA ($RT_{TMBA}$ %) = 84 %

**Revendications**

**1 -** Procédé de préparation de composés aromatiques polyalkoxylés de formule générale (I) :

$$(R_1-O)_m - Ro - (O-R)_n \quad (I)$$

dans laquelle :

– m et n sont des nombres entiers supérieurs ou égaux à 1 avec, de préférence, m + n compris entre 2 et 6,

– $R_o$ représente un radical cyclique aromatique ayant au moins 5 atomes dans le cycle, éventuellement substitué, et représentant au moins l'un des radicaux suivants :

. un radical carbocyclique aromatique, monocyclique ou polycyclique,

. un radical hétérocyclique aromatique, monocyclique ou polycyclique comportant au moins un des hétéroatomes O, N et S,

– R représente un radical hydrocarboné ayant de 1 à 12 atomes de carbone, qui peut être un radical aliphatique acyclique, saturé ou insaturé, linéaire ou ramifié ; un radical cycloaliphatique, saturé ou insaturé, monocyclique ou polycyclique ; un radical cycloaliphatique- ou arylaliphatique, saturé ou insaturé, linéaire ou ramifié,

– $R_1$ est un radical alkyle, linéaire ou ramifié ayant de 1 à 6 atomes de carbone,

procédé caractérisé par le fait que l'on effectue successivement les étapes suivantes :

1°- On fait réagir un composé aromatique porteur d'au moins un atome d'halogène et d'au moins un groupe hydroxyle répondant à la formule générale (II) :

$$(HO)_m - Ro - (X)_n \quad (II)$$

dans laquelle :

– m et n sont des nombres entiers supérieurs ou égaux à 1 avec, de préférence, m + n compris entre 2 et 6,

– X représente un atome d'iode, de brome ou de chlore,

– $R_o$ représente un radical cyclique aromatique ayant au moins 5 atomes dans le cycle, éventuellement substitué, et représentant au moins l'un des radicaux suivants :

. un radical carbocyclique aromatique, monocyclique ou polycyclique,

. un radical hétérocyclique aromatique, monocyclique ou polycyclique comportant au moins un des

hétéroatomes O, N et S,

– et un alcoolate de métal alcalin ou alcalino-terreux de formule générale (III) :

$$m^{w+} [ O - R ]_{w}^{-} \qquad (III)$$

dans laquelle :

– M représente un métal alcalin ou alcalino-terreux,

– w représente la valence du métal alcalin ou alcalino-terreux,

– R représente un radical hydrocarboné ayant de 1 à 12 atomes de carbone, qui peut être un radical aliphatique acyclique, saturé ou insaturé, linéaire ou ramifié ; un radical cycloaliphatique, saturé ou insaturé, monocyclique ou polycyclique ; un radical cycloaliphatique- ou arylaliphatique, saturé ou insaturé, linéaire ou ramifié,

en présence d'un catalyseur au cuivre qui est le cuivre et/ou un composé du cuivre et d'une quantité efficace d'un co-catalyseur choisi parmi les carbonates organiques, les carbonates mixtes organo-métalliques, le dioxyde de carbone ou les composés susceptibles de former du dioxyde de carbone,

2° - On réalise la O-alkylation du ou des groupes hydroxyle du produit obtenu précédemment après alkoxylation, par addition directement dans le milieu réactionnel organique d'un agent d'alkylation choisi parmi les halogénures d'alkyle inférieurs, les dialkylsulfates, les dialkylcarbonates.

**2 -** Procédé selon la revendication 1 caractérisé par le fait que le composé aromatique porteur d'au moins un atome d'halogène et d'au moins un groupe hydroxyle répond à la formule générale (II) dans laquelle $R_o$ symbolise :

1° - un radical carbocyclique aromatique, monocyclique ou polycyclique,

2° - un radical hétérocyclique aromatique, monocyclique ou polycyclique,

3° - un radical divalent constitué par un enchaînement de groupements, tels que définis aux paragraphes 1 et/ou 2 liés entre eux :

. par un lien valentiel,

. par un radical alkylène ou alkylidène ayant de 1 à 4 atomes de carbone, de préférence un radical méthylène ou isopropylidène,

. par l'un des groupes suivants :

$-O-$ ,  $-CO-$ ,

$-S-$ ,  $-SO-$ ,  $-SO_2-$ ,

```
—N— ,    —CO—N—
 |            |
 R2           R2
```

dans ces formules, $R_2$ représentant un atome d'hydrogène ou un radical alkyle ayant de 1 à 4 atomes de carbone, un radical cyclohexyle ou phényle.

**3 -** Procédé selon l'une des revendications 1 et 2 caractérisé par le fait que le composé aromatique porteur d'au moins un atome d'halogène et d'au moins un groupe hydroxyle répond à la formule générale (II) dans laquelle $R_o$ est substitué par un ou plusieurs substituants tels que :

. un radical de formule $-R_3-OH$ dans laquelle le radical $R_3$ représente un lien valentiel ou un radical hydrocarboné divalent, linéaire ou ramifié, saturé ou insaturé ayant de 1 à 4 atomes de carbone, tel que par exemple, méthylène, éthylène, propylène, isopropylène, isopropylidène,

. un radical alkyle, linéaire ou ramifié, ayant de 1 à 6 atomes de carbone, de préférence de 1 à 4 atomes de carbone, tel que méthyle, éthyle, propyle, isopropyle, butyle, isobutyle, secbutyle, tert-butyle,

. un radical alcényle linéaire ou ramifié ayant de 2 à 6 atomes de carbone, de préférence, de 2 à 4 atomes de carbone, tel que vinyle, allyle,

. un radical alkoxy linéaire ou ramifié ayant de 1 à 6 atomes de carbone, de préférence de 1 à 4 atomes de carbone tel que les radicaux méthoxy, éthoxy, propoxy, isopropoxy, butoxy,

. un groupe -CHO,

. un groupe acyle ayant de 2 à 6 atomes de carbone,

. un radical de formule $-R_3-COOH$, $R_3$ ayant la signification donnée précédemment,

. un radical de formule $-R_3-COOR_4$ dans laquelle $R_3$ a la signification donnée précédemment et $R_4$ représente un radical alkyle, linéaire ou ramifié, ayant de 1 à 6 atomes de carbone,

. un radical de formule $-R_3-NH_2$ avec un groupe $NH_2$ N-protégé, $R_3$ ayant la signification donnée précédemment,

. un radical de formule $-R_3-N(R'_4)_2$ dans laquelle $R_3$ a la signification donnée précédemment et les radicaux $R'_4$ identiques ou différents représentent un atome d'hydrogène ou un radical alkyle, linéaire ou ramifié, ayant de 1 à 6 atomes de carbone,

. un radical de formule $-R_3-CO-N(R'_4)_2$, $R_3$ et $R'_4$ ayant la signification donnée précédemment,

. un radical de formule $-R_3-Z$ dans laquelle $R_3$ a la signification donnée précédemment et Z symbolise un atome d'halogène X, tel que précédemment défini, un atome de fluor ou un groupe $CF_3$.

**4 -** Procédé selon l'une des revendications 1 à 3 caractérisé par le fait que le composé aromatique porteur d'au moins un atome d'halogène et d'au moins un groupe hydroxyle répond à la formule générale (II) est choisi parmi :

- le bromo-2 phénol
- le bromo-3 phénol
- le bromo-4 phénol
- le bromo-1 naphtol-2
- le bromo-6 naphtol-2
- le bromo-2 méthyl-4 phénol
- le bromo-4 diméthyl-2,6 phénol
- le bromo-4 diméthyl-3,5 phénol
- le dibromo-2,6 méthyl-4 phénol
- le bromo-2-p-crésol
- le bromo-2 chloro-4 phénol
- le bromo-4 chloro-2 phénol
- le bromo-4 chloro-6-o-crésol
- les bromofluorophénols
- les bromo bis-phénols
- les bromo isopropylidène-4,4' bis-phénols

**5 -** Procédé selon l'une des revendications 1 à 4 caractérisé par le fait qu'il comporte les étapes suivantes :

1° - On fait réagir un halogéno hydroxy benzaldéhyde répondant à la formule générale (IIa) :

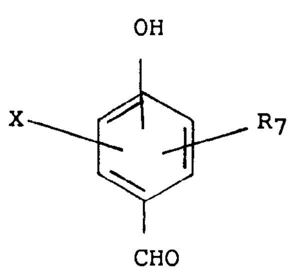

(IIa)

dans laquelle :

- X représente un atome d'iode, de brome ou de chlore,
- $R_7$ représente un atome d'hydrogène, un atome d'iode, de brome, un atome de chlore, un radical alkyle ayant de 1 à 4 atomes de carbone, un radical alcoxy ayant de 1 à 4 atomes de carbone, un radical hydroxyle,

dans ladite formule (IIa), le radical hydroxyle pouvant être en position ortho-, méta- ou para de la fonction aldéhyde,

avec un alcoolate de métal alcalin ou alcalino-terreux ayant de 1 à 4 atomes de carbone, en présence d'un catalyseur au cuivre qui est le cuivre et/ou un composé du cuivre et d'une quantité efficace d'un co-catalyseur choisi parmi les carbonates organiques, les carbonates mixtes organométalliques, le dioxyde de carbone ou les composés susceptibles de former du dioxyde de carbone,

2° **-** On réalise la O-alkylation du ou des groupes hydroxyle du produit obtenu précédemment après alkoxylation, par addition directement dans le milieu réactionnel organique d'un agent d'alkylation apportant le reste alkyle $R'_1$ ayant de 1 à 4 atomes de carbone.

**6 -** Procédé selon la revendication 5 caractérisé par le fait que l' halogéno hydroxy benzaldéhyde répond à l'une des formules suivantes :

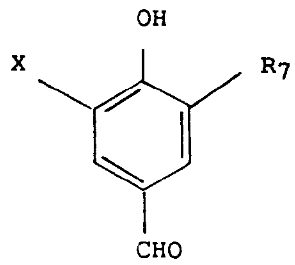

(IIa$_1$)

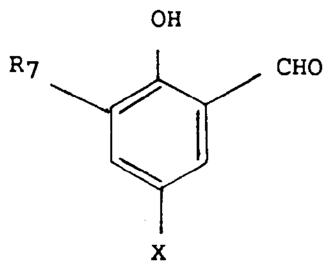

(IIa$_2$)

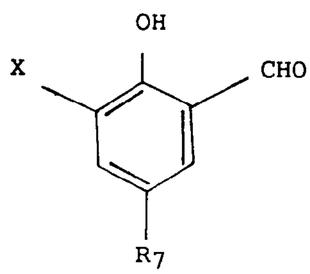

(IIa$_3$)

dans lesquelles :

– X et $R_7$ ont les significations données précédemment.

**7 -** Procédé selon l'une des revendications 1 à 6 caractérisé par le fait que l'halogéno hydroxy benzaldéhyde est choisi parmi :

– le bromo-3 hydroxy-4 benzaldéhyde,
– le iodo-3 hydroxy-4 benzaldéhyde
– le dibromo-3,5 hydroxy-4 benzaldéhyde
– le diiodo-3,5 hydroxy-4 benzaldéhyde
– le bromo-5 méthoxy-3 hydroxy-4 benzaldéhyde
– le iodo-5 méthoxy-3 hydroxy-4 benzaldéhyde
– le bromo-5 éthoxy-3 hydroxy-4 benzaldéhyde
– le iodo-5 éthoxy-3 hydroxy-4 benzaldéhyde
– le bromo-3 dihydroxy-4,5 benzaldéhyde
– le iodo-3 dihydroxy-4,5 benzaldéhyde
– le bromo-3 dihydroxy-2,5 benzaldéhyde
– le iodo-3 dihydroxy-2,5 benzaldéhyde
– le bromo-2 hydroxy-4 benzaldéhyde
– le iodo-2 hydroxy-4 benzaldéhyde
– le bromo-4 hydroxy-3 benzaldéhyde
– le iodo-4 hydroxy-3 benzaldéhyde
– le bromo-3 hydroxy-2 benzaldéhyde
– le iodo-3 hydroxy-2 benzaldéhyde
– le bromo-5 hydroxy-2 benzaldéhyde
– le iodo-5 hydroxy-2 benzaldéhyde

**8 -** Procédé selon l'une des revendications 1 à 7 caractérisé par le fait que l'alcoolate de métal alcalin est un alcoolate de sodium ou de potassium d'un alcanol primaire ou secondaire ayant 1 à 4 atomes de carbone, de préférence, le méthylate de sodium ou l'éthylate de sodium.

**9 -** Procédé selon l'une des revendications 1 à 8 caractérisé par le fait que le catalyseur est un composé

organique ou inorganique du cuivre I ou du cuivre II, choisi parmi le chlorure cuivreux, le chlorure cuivrique, le carbonate de cuivre II basique, le nitrate cuivreux, le nitrate cuivrique, le sulfate cuivrique, l'acétate cuivrique, le trifluorométhylsulfonate cuivrique, l'hydroxyde cuivrique, le picolinate de cuivre II, le méthylate de cuivre I, le méthylate de cuivre II, le chélate de cuivre II de la 8-quinoléine, les composés de cuivre de formule $ClCuOCH_3$, $Cu_2(OCH_3)_2(acac)_2$.

**10 -** Procédé selon l'une des revendications 1 à 9 caractérisé par le fait que le co-catalyseur est un carbonate de formule générale (IV) :

$$R_8 - O - CO - O - R_9 \qquad (IV)$$

dans laquelle :

– $R_8$ représente :

. un radical alkyle, linéaire ou ramifié, ayant 1 à 6 atomes de carbone,

. un radical cycloalkyle ayant 5 à 6 atomes de carbone,

. un radical cycloalkyle, ayant 5 ou 6 atomes de carbone substitué par un ou deux radicaux alkyle ayant 1 à 4 atomes de carbone,

. un radical phényle,

. un radical phényle substitué par 1 ou 2 radicaux alkyle ayant 1 à 4 atomes de carbone,

. un radical $R_{10}$ - O - CO - dans lequel $R_{10}$ représente un radical alkyle, linéaire ou ramifié ayant 1 à 6 atomes de carbone, un radical phényle, un radical cycloalkyle ayant 5 ou 6 atomes de carbone.

– $R_9$ représente :

. un radical alkyle, linéaire ou ramifié, ayant 1 à 6 atomes de carbone,

. un radical cycloalkyle ayant 5 ou 6 atomes de carbone,

. un radical cycloalkyle ayant 5 ou 6 atomes de carbone substitué par un ou deux radicaux alkyle ayant 1 à 4 atomes de carbone,

. un radical phényle,

. un radical phényle substitué par un ou deux radicaux alkyle ayant 1 à 4 atomes de carbone,

. un atome de sodium ou de potassium,

– $R_8$ et $R_9$ peuvent former ensemble un radical alkylène ayant 2 à 6 atomes de carbone

**11 -** Procédé selon l'une des revendications 1 à 10 caractérisé par le fait que le co-catalyseur est un carbonate de formule générale (IVa) :

$$R_8 - O - CO - R_9 \qquad (IV_a)$$

dans laquelle :

– $R_8$ représente :

. un radical alkyle, linéaire ou ramifié, ayant 1 à 6 atomes de carbone,

. un radical cycloalkyle ayant 5 à 6 atomes de carbone,

. un radical cycloalkyle, ayant 5 ou 6 atomes de carbone substitué par un ou deux radicaux alkyle ayant 1 à 4 atomes de carbone,

. un radical phényle,

. un radical phényle substitué par 1 ou 2 radicaux alkyle ayant 1 à 4 atomes de carbone,

. un radical $R_{10}$ - O - CO - dans lequel $R_{10}$ représente un radical alkyle, linéaire ou ramifié ayant 1 à 6 atomes de carbone, un radical phényle, un radical cycloalkyle ayant 5 ou 6 atomes de carbone

– $R_9$ représente :

. un radical alkyle, linéaire ou ramifié, ayant 1 à 6 atomes de carbone,

. un radical cycloalkyle ayant 5 ou 6 atomes de carbone,

. un radical cycloalkyle ayant 5 ou 6 atomes de carbone substitué par un ou deux radicaux alkyle ayant 1 à 4 atomes de carbone,

. un radical phényle,

. un radical phényle substitué par un ou deux radicaux alkyle ayant 1 à 4 atomes de carbone,

. un atome de sodium ou de potassium,

– $R_8$ et $R_9$ peuvent former ensemble un radical alkylène ayant 2 à 6 atomes de carbone.

**12 -** Procédé selon l'une des revendications 1 à 11 caractérisé par le fait que le co-catalyseur est le dioxyde de carbone.

**13 -** Procédé selon l'une des revendications 1 à 12 caractérisé par le fait que l'alcoolate de métal alcalin ou alcalino-terreux est mis en oeuvre en une quantité correspondant à la quantité stoechiométrique nécessaire pour salifier les groupes hydroxyle plus une quantité égale de 1 fois à 5 fois, et de préférence de 1 à 3 fois, la quantité stoechiométrique nécessaire pour transformer le ou les atomes d'halogène en groupements alkoxy.

**14 -** Procédé selon la revendication 1 à 13 caractérisé par le fait que la concentration de l'alcoolate de métal alcalin ou alcalino-terreux est supérieure à 1 mole/litre et, de préférence comprise entre 1 et 5 moles/litre.

**15 -** Procédé selon l'une des revendications 1 à 14 caractérisé par le fait que le rapport molaire composé

du cuivre/composé de formule (II) est de 1 % à 50 % et, de préférence, de 2 % à 20 % et que le rapport molaire co-catalyseur/composé du cuivre est de 1 à 10 et, de préférence, de 1 à 5.

**16 -** Procédé selon l'une des revendications 1 à 15 caractérisé par le fait que la réaction est conduite dans un solvant constitué par l'alcanol correspondant à l'alcoolate de métal alcalin utilisé.

**17 -** Procédé selon l'une des revendications 1 à 16 caractérisé par le fait que l'agent alkylation dans la réaction de O-alkylation est :

– le diméthylsulfate ou le diméthylcarbonate.

– un halogénure d'alkyle inférieur répondant à la formule générale (v) :

$$R_1\text{-}X \qquad (V)$$

dans ladite formule, X représente un atome de brome, de chlore ou d'iode et $R_1$ représente un radical alkyle linéaire ou ramifié ayant de 1 à 6 atomes de carbone, de préférence, le chlorure de méthyle, le chloroéthane, le bromure de méthyle et le bromoéthane.

**18 -** Procédé selon l'une des revendications 1 à 17 caractérisé par le fait que la quantité d'halogénure d'alkyle exprimée par rapport à la quantité de composé de formule (II) varie de la quantité stoechiométrique à un excès pouvant atteindre 200 %, de préférence égale à la quantité stoechiométrique.

**19 -** Procédé selon l'une des revendications 1 à 18 caractérisé par le fait que l'on introduit avant l'agent d'alkylation, un sel sous forme d'iodure de métal alcalin, de préférence de sodium.

**20 -** Procédé selon l'une des revendications 1 à 19 caractérisé par le fait que le composé aromatique polyalkoxylé de formule (I) est l'un des composés suivants :

– le diméthoxy-1,2 benzène
– le triméthoxy-3,4,5 benzaldéhyde
– le diméthoxy-3,4 benzaldéhyde
– le diméthoxy-2,5 benzaldéhyde
– le diéthoxy-3,4 benzaldéhyde
– l'éthoxy-3 méthoxy-4 benzaldéhyde

Office européen des brevets

# RAPPORT DE RECHERCHE EUROPEENNE

Numero de la demande

EP 91 40 1683

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5) |
|---|---|---|---|
| A | EP-A-0 277 894 (RHONE-POULENC CHIMIE)<br>* Exemples; revendications *<br>--- | 1-20 | C 07 C 47/575<br>C 07 C 45/64<br>C 07 C 41/16 |
| A | EP-A-0 155 335 (LUDWIG HEUMANN)<br>* Exemples; revendications *<br>--- | 1-20 | |
| A | GB-A-2 089 672 (STERWIN)<br>* Revendications; page 5, lignes 1-25 *<br>--- | 1-20 | |
| A | SYNTHESIS, 1983, page 308; D.V. RAO et al.: "An efficient synthesis of 3,4,5-trimethoxybenzaldehyde from vanillin"<br>* Page 308 *<br>--- | 1-20 | |
| A | DE-A-1 905 823 (SHELL INTERNATIONALE RESEARCH)<br>* Revendication 1 *<br>--- | 1-20 | |
| P,X | EP-A-0 423 010 (RHONE-POULENC CHIMIE)<br>* Revendications; exemples *<br>----- | 1-20 | DOMAINES TECHNIQUES RECHERCHES (Int. Cl.5)<br>C 07 C 41/00<br>C 07 C 45/00<br>C 07 C 47/00 |

Le présent rapport a été établi pour toutes les revendications

| Lieu de la recherche | Date d'achèvement de la recherche | Examinateur |
|---|---|---|
| LA HAYE | 02-10-1991 | WRIGHT M.W. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P0402)